(19) Europäisches Patentamt
European Patent Office
Office européen des brevets

(11) Veröffentlichungsnummer : **0 053 247**
**B1**

(12) # EUROPÄISCHE PATENTSCHRIFT

(45) Veröffentlichungstag der Patentschrift : **18.01.84**

(51) Int. Cl.³ : **C 07 C121/78**, C 07 C120/00, C 07 C101/54

(21) Anmeldenummer : **81108202.3**

(22) Anmeldetag : **12.10.81**

(54) **Verfahren zur Herstellung von 6-Fluor-anthranilsäurenitril oder 6-Fluor-anthranilsäure und 6-Fluor-anthranilsäurenitril.**

(30) Priorität : **28.11.80 DE 3044904**

(43) Veröffentlichungstag der Anmeldung : **09.06.82 Patentblatt 82/23**

(45) Bekanntmachung des Hinweises auf die Patenterteilung : **18.01.84 Patentblatt 84/03**

(84) Benannte Vertragsstaaten : **BE CH DE FR GB LI**

(56) Entgegenhaltungen :
**FR-A- 2 321 485**
**CHEMICAL ABSTRACTS, Band 81, Nr. 15, 14. Oktober 1974, Seite 433, Nr. 91190y Columbus, Ohio, U.S.A. E.M. GRIVSKY et al.: "Syntheses of 2-chloro-4-acetylaminobenzonitrile isomers and structurally related compounds with biological activities"**
**METHODEN DER ORGANISCHEN Chemie, Band 5/3, Houben-Weyl, 1962 Georg Thieme Verlag Stuttgart, DE. "Halogen-verbindungen, Fluorverbindungen", Seiten 409-415**

(73) Patentinhaber : **BASF Aktiengesellschaft**
**Carl-Bosch-Strasse 38**
**D-6700 Ludwigshafen (DE)**

(72) Erfinder : **Jacobs, Peter, Dr.**
**Kalmitstrasse 2**
**D-6718 Gruenstadt (DE)**
Erfinder : **Oeser, Heinz-Guenter, Dr.**
**Mohngasse 25**
**D-6711 Dirmstein (DE)**

**EP 0 053 247 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von 6-Fluor-anthranilsäurenitril oder 6-Fluor-anthranilsäure und 6-Fluor-anthranilsäurenitril

Die Erfindung betrifft ein Verfahren zur Herstellung von 6-Fluor-anthranilsäurenitril oder 6-Fluor-anthranilsäure durch Umsetzung von 2,6-Difluorbenzonitril mit Ammoniak und gewünschtenfalls durch Verseifung des resultierenden 6-Fluoranthranilsäurenitrils mit basischen Verbindungen sowie 6-Fluor-anthranilsäurenitril.

Es ist aus Houben-Weyl, Methoden der Organischen Chemie, Band 5/3, Seiten 409 bis 415 bekannt, daß Fluor in aromatischen Verbindungen auf verschiedene Weise substituiert werden kann, wobei die Substituierung an nichtsubstituierten Aromaten schwierig ist, bei entsprechender Substituierung durch aktivierende Stoffe wie die Nitrogruppe, schon vorhandene Chlor- oder Fluoratome die Ausbeute erhöht wird. Houben-Weyl zeigt, daß je nach Art von weiteren Substituenten Fluor leichter oder schwerer substituiert wird und eine genauere Voraussage über Substituierungen und Stellung der Substituenten schwierig ist.

Es wurde nun gefunden, daß man 6-Fluor-anthranilsäurenitril oder 6-Fluoranthranilsäure der Formel

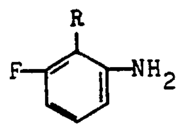

(I)

worin R den Rest —CN oder —COOH bedeutet, vorteilhaft herstellt, wenn man

a) 2,6-Difluorbenzonitril der Formel

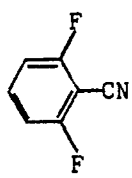

(II)

mit Ammoniak umsetzt und dann gewünschtenfalls

b) das resultierende 6-Fluor-anthranilsäurenitril der Formel

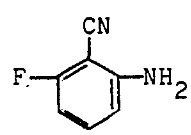

(III)

mit einer basischen Verbindung umsetzt.

Weiterhin wurde die neue Anthranilsäureverbindung der Formel III gefunden.

Die Umsetzung kann durch die folgenden Formeln wiedergegeben werden :

a) 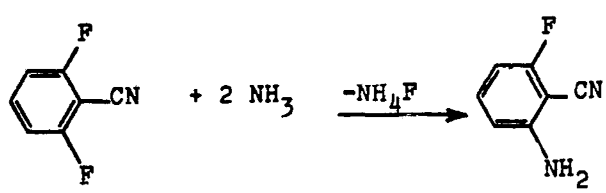


(II) (III)

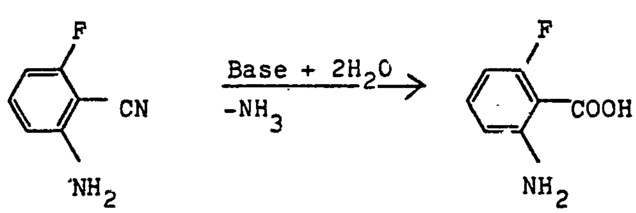


(III) (I)

Im Hinblick auf den Stand der Technik liefert das Verfahren nach der Erfindung auf einfacherem und wirtschaftlicherem Wege 6-Fluor-anthranilsäurenitril und 6-Fluor-anthranilsäure in guter Ausbeute und Reinheit. Diese vorteilhaften Ergebnisse sind überraschend. Es war zu erwarten, daß bei dem erfindungsgemäßen Verfahren durch die Bildung von z. B.

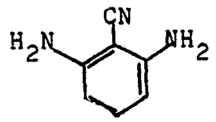
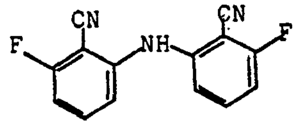
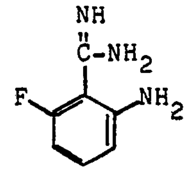

und ihren Folgeprodukten nur geringe Ausbeuten an Nitril I gebildet werden und inhomogene Gemische entstehen.

Die Verbindung II kann nach bekannten Verfahren, z. B. durch Umsetzung von 2,6-Dichlorbenzonitril mit Kaliumfluorid nach der in DE-OS 28 03 259 beschriebenen Arbeitsweise hergestellt werden. Im Schritt a) kann Ammoniak mit dem Ausgangsstoff II in stöchiometrischer Menge oder im Überschuß, vorteilhaft in einem Verhältnis von 2 bis 10, insbesondere 2 bis 5 Mol Ammoniak je Mol Ausgangsstoff II umgesetzt werden. Ammoniak kann gasförmig, in Gestalt wäßriger, zweckmäßig 10- bis 25-gewichtsprozentiger Ammoniaklösungen oder vorteilhaft flüssig verwendet werden.

Die Umsetzung a) wird im allgemeinen bei einer Temperatur von 0 bis 200, zweckmäßig 50 bis 150, insbesondere 70 bis 120 °C drucklos oder unter Druck, zweckmäßig unter dem sich bei der Reaktion einstellenden Druck, kontinuierlich oder diskontinuierlich durchgeführt.

Die Umsetzung kann wie folgt durchgeführt werden : Ein Gemisch von Verbindung II mit oder ohne Lösungsmittel (z. B. Alkanole wie Ethanol) und Ammoniak wird während 0,5 bis 11 Stunden bei der Reaktionstemperatur gehalten. Dann wird der Endstoff mit den üblichen Mitteln, z. B. gegebenenfalls Zugabe eines geeigneten Lösungsmittels wie Halogenkohlenwasserstoffen, z. B. Dichlormethan, Absaugen, Wäsche und Trocknung der gebildeten organischen Phase und Destillation, isoliert.

Die Verseifung der Verbindung II zur 6-Fluor-anthranilsäure I erfolgt in der Regel in alkalischem Medium, mit Erdalkali- oder Alkaliverbindungen, vorzugsweise in wäßrigen Lösungen, zweckmäßig 10- bis 50-gewichtsprozentige Lösungen von Erdalkali- oder Alkalihydroxiden. Bevorzugt sind Natron- und Kalilauge. Man verwendet z. B. die Verbindung III 5- bis 30-gewichtsprozentig in solchen Lösungen und verseift zweckmäßig bei einem pH von 7 bis 14, vorzugsweise 8 bis 13 und bei einer Temperatur von 0 bis 150 °C, bevorzugt von 15 bis 30 °C, während 6 bis 15 Stunden. Vorteilhaft ist ein Zusatz von Alkohol, im allgemeinen Alkanolen von zweckmäßig 1 bis 6 Kohlenstoffatomen, vorzugsweise in einer Menge von 2 bis 10, insbesondere 4 bis 8 Gewichtsprozent Alkohol, bezogen auf die Wassermenge.

Die Reaktion kann wie folgt durchgeführt werden : Ein Gemisch von Verbindung III, wäßriger Alkalilauge und Alkohol wird während 0,5 bis 9 Stunden bei der Verseifungstemperatur gehalten. Der Endstoff wird dann mit üblichen Mitteln, z. B. Ansäuern auf pH 3 bis 4, Absaugen, Waschen des Feststoffs, Extraktion der wäßrigen Phase, z. B. in einem geeigneten Lösungsmittel wie Dichlormethan, Destillation, abgetrennt.

Die beiden Verbindungen I sind wertvolle Zwischenprodukte zur Herstellung von Verbindungen, welche sich zur Bekämpfung von verschiedenartigen tierischen und pflanzlichen Schädlingen eignen. So können sie folgender Umsetzung unterworfen werden :

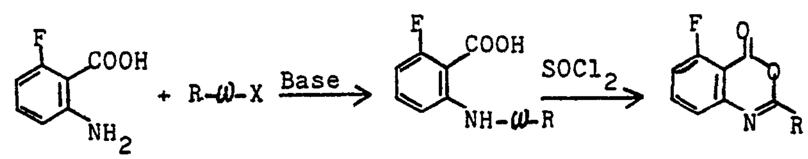


X = Halogen

wobei man wertvolle Wirkstoffe erhält.

Die in den folgenden Beispielen genannten Teile bedeuten Gewichtsteile.

Beispiel 1

6-Fluor-anthranilsäurenitril

50 Teile 2,6-Difluorbenzonitril und 40 Volumenteile Ammoniak werden in einem geschlossenen Gefäß während 10 Stunden bei 100 °C gerührt. Das Reaktionsgemisch wird in Dichlormethan aufgenommen. Der Feststoff abgesaugt, die organische Phase dreimal mit Wasser ausgeschüttelt, über Natriumsulfat getrocknet, das Lösungsmittel abgezogen und der Rückstand im Vakuum getrocknet. Man erhält 48 Teile 6-Fluor-anthranilsäurenitril vom Festpunkt 125 bis 128 °C (98,1 % der Theorie).

IR (KBr) : 3 450, 3 360 ($NH_2$), 2 215 $cm^{-1}$ (CN) $^1H$-NMR ($CDCl_3$) : = 6,45 (s, 2H, $NH_2$), 6,35-6,74 (m, 2H, Arom.), 7,19-7,49 (m, 1H, Arom.).

Beispiel 2

1 000 Teile 2,6-Difluorbenzonitril, 1 500 Volumenteile Tetrahydrofuran und 800 Volumenteile Ammoniak werden in einem geschlossenen Gefäß 10 Stunden bei 100 °C gerührt. Es wird auf Raumtemperatur abgekühlt, der Feststoff abgesaugt, mit Tetrahydrofuran nachgewaschen und die organische Phase im Vakuum vom Lösungsmittel befreit. Der Rückstand wird im Vakuum getrocknet. Man erhält 972 Teile 6-Fluor-anthranilsäurenitril vom Festpunkt 127 °C (99 % der Theorie).

Beispiel 3

6-Fluor-anthranilsäure

56 Teile 6-Fluor-anthranilsäurenitril, 33 Teile Natriumhydroxid, 250 Teile Wasser und 20 Volumenteile Ethanol werden 8 Stunden am Rückfluß erwärmt. Anschließend wird mit 20-gewichtsprozentiger, wäßriger Schwefelsäure auf pH 3 bis 4 eingestellt, der ausgefallene Niederschlag abgesaugt, mit Wasser gewaschen und getrocknet. Die wäßrige Phase wird mit Dichlormethan extrahiert, das Lösungsmittel im Vakuum abgezogen und der Rückstand ebenfalls im Vakuum getrocknet. Man erhält so insgesamt 58 Teile 6-Fluor-anthranilsäure vom Festpunkt 165 bis 7 °C (90,9 % der Theorie).

**Ansprüche**

1. Verfahren zur Herstellung von 6-Fluor-anthranilsäurenitril oder 6-Fluor-anthranilsäure der Formel

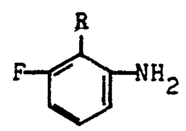

(I)

worin R den Rest —COOH oder —CN bedeutet, dadurch gekennzeichnet, daß man

a) 2,6-Difluorbenzonitril der Formel

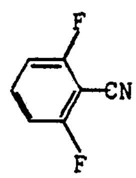

(II)

mit Ammoniak umsetzt und dann gewünschtenfalls

b) das resultierende 6-Fluor-anthranilsäurenitril der Formel

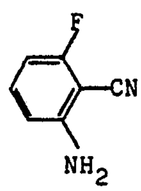

(III)

mit einer basischen Verbindung umsetzt.

2. 6-Fluor-anthranilsäurenitril der Formel

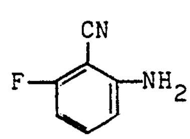

(III)

**Claims**

1. A process for the preparation of 6-fluoroanthranilonitrile or 6-fluoroanthranilic acid of the formula

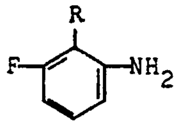

(I)

where R is —COOH or —CN respectively, wherein

a) 2,6-difluorobenzonitrile of the formula

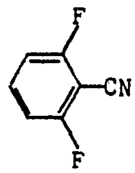

(II)

is reacted with ammonia and thereafter, if desired,

b) the resulting 6-fluoroanthranilonitrile of the formula

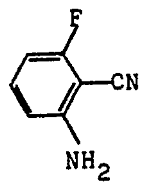

(III)

is reacted with a basic compound.

2. 6-Fluoroanthranilonitrile of the formula

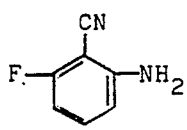

(III)

**Revendications**

1. Procédé de préparation du nitrile de l'acide fluoro-6 anthranilique ou de l'acide fluoro-6 anthranilique de la formule

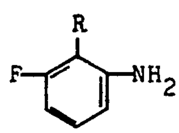

(I)

dans laquelle R désigne un groupe —COOH ou —CN, caractérisé en ce que l'on fait réagir

a) le difluoro-2,6 benzonitrile de la formule

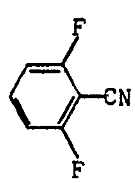

(II)

avec l'ammoniac, puis, le cas échéant,

b) le nitrile de l'acide fluoro-6 anthranilique formé, de la formule

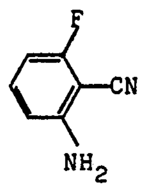

(III)

avec une substance basique.

2. Le nitrile de l'acide fluoro-6 anthranilique de la formule

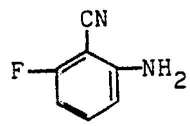

(III)